# FASCICULE DE BREVET EUROPEEN

(11) **EP 1 795 175 B1**
(45) Date de publication et mention de la délivrance du brevet: **11.04.2012**
(21) Numéro de dépôt: 06124930.6
(22) Date de dépôt: 28.11.2006
(51) Int. Cl.: A61K 8/02, A61Q 1/14, A61Q 19/10

(54) **Article cosmétique soluble à effet thermique**
Löslicher kosmetischer Artikel mit thermischen Effekt
Soluble cosmetic article with thermal effect

(30) Priorité: 07.12.2005 FR 0553750
(43) Date de publication de la demande: 13.06.2007
(73) Titulaire: L'Oréal, 75008 Paris (FR)
(72) Inventeur: Aubrun-Sonneville, Odile, 92160 Antony (FR)
(74) Mandataire: Duvert, Sandra

(56) Documents cités:
- EP-A- 0 750 905
- EP-A- 1 172 088
- WO-A-2005/003423
- GB-A- 2 302 651
- US-A- 4 416 791

## Description

La présente invention concerne un article cosmétique à effet thermique, comprenant au moins un support soluble dans l'eau et au moins un composé à réaction endothermique ou exothermique.

Les effets thermiques sont généralement utilisés dans le domaine du soin de la peau pour compléter et /ou amplifier l'efficacité des produits.

La chaleur sur la peau provoque l'ouverture des pores, ce qui améliore l'efficacité d'une composition cosmétique appliquée sur la peau. Ainsi, les effets chauffants peuvent être utilisés notamment dans des produits de nettoyage profond, tels que les produits exfoliants contenant des scrubs, ou aussi dans des produits relaxants. La chaleur vient renforcer la sensation de nettoyage profond ou de détente. Ces effets chauffants peuvent également être utilisés en association avec des actifs ou agents cosmétiques qui sont rendus plus actifs sous l'effet de la chaleur.

Les effets froids sont généralement utilisés dans des produits nettoyants dont on veut renforcer la valence fraîcheur et l'effet tonifiant. Ils peuvent également être utilisés dans des produits de soin, notamment des produits hydratants pour renforcer la sensation d'hydratation.

En général, ces effets thermiques sont obtenus grâce à des composés exothermiques ou endothermiques utilisés tels quels ou plus généralement utilisés dans des compositions anhydres contenant des composés exothermiques ou endothermiques. Ces compositions anhydres contiennent le plus souvent une forte quantité d'huiles ou de polyols et notamment de glycols.

Ainsi, le document DE-A-10009252 décrit des gels nettoyants contenant au moins 40 % de polyols et des sels hydrosolubles en particules. Le document EP-A-1106164 décrit des compositions cosmétiques solides, comprenant une poudre à base de particules solides de polymère expansé et un liant contenant de l'huile et un ou plusieurs agents susceptibles de dégager de la chaleur tels que polyols et zéolithe. Le document EP-A-966956 décrit des compositions anhydres pulvérulentes contenant une poudre à base de particules solides de polymère expansé et un liant contenant un ou plusieurs agents susceptibles de dégager de la chaleur, tels que polyols et zéolithe.

Toutefois, ces compositions présentent des propriétés peu cosmétiques du fait des taux élevés de polyols qui rendent les compositions collantes et lourdes, ou des taux élevés d'huiles, qui conduisent à un effet gras important. En outre, il est très souvent utile d'épaissir ces milieux de manière à mettre en suspension les sels ou les zéolithes nécessaires à l'obtention de l'effet thermique. Cependant, cette opération est rendue difficile en raison du mauvais gonflement des polymères gélifiants dans ces milieux.

Par ailleurs, ces compositions peuvent imposer des contraintes de formulation en limitant le domaine de formulation aux composants solubles ou dispersibles dans les glycols ou les huiles. Ces inconvénients sont parfois contournés en utilisant des articles de conditionnement bicompartimentés permettant de séparer la composition chauffante des autres ingrédients utiles à la composition mais incompatibles avec la composition chauffante. On essaie d'éviter l'emploi de ces conditionnements complexes.

Les documents EP-A-1172088 et GB-A-2302651 décrivent des compositions exothermiques imprégnés sur des substrats insolubles.

Le document WO-A-2005/003423 décrit un support fibreux soluble dans l'eau imprégné avec une composition.

Le document US-A-4416791 décrit une composition détergente contenue dans la cavité formée par un film hydrosoluble.

Le document EP-A-750905 décrit des lignettes comprenant une couche hydrosoluble et un composé à effet thermique.

Il subsiste donc le besoin de compositions à effet thermique, n'ayant pas les inconvénients de celles de l'art antérieur, et notamment présentant de bonnes propriétés cosmétiques tout en donnant l'effet thermique, chaud ou froid, recherché.

La présente demande répond à ce besoin. En effet, la demanderesse a trouvé de manière surprenante qu'il était possible d'inclure des composés à effet thermique dans des supports solubles, donnant des articles qui, humidifiés ou dissous dans l'eau au moment de l'utilisation, donnaient des compositions à effet thermique, ayant de bonnes propriétés cosmétiques.

L'utilisation de ce type d'articles permet de lever les contraintes de formulation dans la mesure où ces articles peuvent conduire à une large gamme de produits, des gels aux crèmes, pour différentes applications, en fonction de la composition contenant le composé à effet thermique, appliquée sur le support. En outre, ils sont faciles à fabriquer.

Ainsi, l'invention réside, selon l'un de ses aspects, dans un article cosmétique ou dermatologique comportant :
- un support sous forme d'au moins une nappe comprenant des fibres solubles dans l'eau à une température inférieure ou égale à 30°C, ledit support présentant une densité inférieure ou égale à 0,1 g/cm³, et
- une composition portée par le support, contenant au moins un composé à effet thermique.

Le ou les composés à effet thermique peuvent être incorporés tels quels sur le support mais ils peuvent aussi être incorporés en mélange avec d'autres composés. La composition portée par le support peut donc ne comprendre que le ou les composés à effet thermique qui constituent à eux seuls la composition, ou bien elle peut contenir le ou les composés à effet thermique en mélange avec d'autres composés. Cette composition est généralement anhydre, en entendant ici par « anhydre » une composition qui contient une quantité d'eau inférieure ou égale à 1 %, allant donc de 0 à 1 % du poids de la composition. Cette composition et le ou les composés à effet thermique peuvent se présenter avantageusement sous forme de poudre ou de granulés ou éventuellement sous forme pâteuse. Cette composition constitue notamment une composition cosmétique ou dermatologique.

On entend dans la présente demande par « portée par le support », le fait que la composition peut être soit mise sur le support soit introduite dans la cavité formée par le support quand celui-ci comporte au moins deux nappes de fibres.

On entend par « température inférieure ou égale à 30°C », une température ne dépassant pas 30°C mais n'étant pas inférieure à 0°C, par exemple allant de plus de 0°C à 30 °C, mieux de 5 °C à 30 °C et encore mieux de 10°C à 30 °C ou 10°C à 20 °C.

Les termes « nappe » et « couche » doivent être considérés comme synonymes dans la présente demande. Le support de la présente invention se présente sous forme d'une ou plusieurs nappes de fibres, ce qui est différent des films fins hydrosolubles qui ne sont pas sous forme de nappes de fibres. Par rapport à ces films fins hydrosolubles, les supports à base de nappes de fibres hydrosolubles selon l'invention présentent l'avantage de permettre l'incorporation de constituants incompatibles, d'être plus simples à mettre oeuvre car ils ne nécessitent pas de prémélange ni de mise en solution des composants, ni de chauffage pour évaporer le solvant, le procédé étant plus rapide et moins coûteux. En outre, les supports selon l'invention ont l'avantage de permettre une plus grande diversité dans le choix de la forme et de l'aspect de l'article car la nappe de fibres peut avoir une épaisseur et une densité variables donnant accès à une grande variété de forme et de taille sans que cela ne pose des problèmes particuliers, alors que le film fin est difficile à sécher si l'épaisseur est trop grande, et il est fragile et difficile à manipuler si la taille est trop grande.

Selon l'invention l'article se présente sous la forme d'un support comportant au moins deux nappes définissant entre elles une cavité, l'une au moins des nappes comprenant des fibres solubles dans l'eau à une température inférieure ou égale à 30°C, ledit support présentant une densité inférieure ou égale à 0,1 g/cm³,
- la cavité contenant une composition contenant au moins un composé à effet thermique.

Les nappes sont assemblées à leur périphérie et forment ainsi une cavité permettant d'introduire la composition contenant le composé à effet thermique.

Les nappes peuvent être formées entièrement de fibres solubles dans l'eau ou bien l'une des nappes peut être constituée entièrement de fibres solubles et l'autre nappe peut être constituée de fibres insolubles ou à la fois de fibres solubles et de fibres insolubles dans l'eau, ou bien les deux nappes peuvent être constituées à la fois de fibres solubles et de fibres insolubles.

Selon un mode préféré de réalisation, au moins une des nappes est constituée exclusivement de fibres solubles dans l'eau.

Par humidification ou dissolution de l'article selon l'invention dans l'eau ou dans une composition aqueuse, on obtient une composition pour application topique, notamment cosmétique ou dermatologique, donnant un effet thermique.

L'invention a encore pour objet, selon un autre de ses aspects, une composition pour application topique, obtenue par la dissolution dans l'eau, d'un article tel que défini ci-dessus, c'est-à-dire une composition obtenue par dissolution dans l'eau, d'un support sous forme d'au moins une nappe comprenant des fibres solubles dans l'eau à une température inférieure ou égale à 30°C et présentant une densité inférieure ou égale à 0,1 g/cm³, le dit support portant une composition contenant au moins un composé à effet thermique. La composition obtenue par dissolution de l'article peut être obtenue à partir d'un support comprenant une ou plusieurs couches de fibres. La température de dissolution de l'article dans l'eau est généralement la température ambiante (20 à 30°C) mais peut être supérieure à la température ambiante si l'on le souhaite selon l'utilisation envisagée.

L'invention a aussi pour objet, selon un autre de ses aspects, un procédé de traitement cosmétique d'une matière kératinique telle que la peau, les cheveux, les muqueuses et les phanères, et notamment pour le traitement cosmétique de la peau, comportant :
- la formation d'une composition cosmétique par dissolution dans l'eau, d'un support comportant au moins une nappe comprenant des fibres solubles dans l'eau à une température inférieure ou égale à 30°C, ledit support présentant une densité inférieure ou égale à 0,1 g/cm³ et portant au moins un composé à effet thermique,
- l'application de la composition ainsi formée sur la matière kératinique.

Le traitement cosmétique comprend aussi bien le soin que le maquillage.

Par «dissolution dans l'eau à une température inférieure ou égale à 30°C », il faut comprendre une solubilisation dans l'eau à une température allant jusqu'à 30°C avec l'aide d'une agitation manuelle et/ou d'une friction du support le cas échéant, dans un laps de temps typiquement inférieur à 5 mn, de préférence inférieur à 1 mn, de préférence inférieur à 30 secondes. L'invention n'exclut pas qu'une eau de température supérieure à 30 °C soit utilisée pour dissoudre le support.

L'article selon l'invention étant destinée à une application topique, il comprend un milieu physiologiquement acceptable. On entend par « milieu physiologiquement acceptable » un milieu compatible avec les matières kératiniques telles que la peau, les lèvres, les ongles, le cuir chevelu et/ou les cheveux. Il en est de même du support, ainsi que de la composition portée par le support.

L'article selon l'invention ne contient pas d'adhésif, mais il peut adhérer à la peau quand il est humidifié.

Cet article est flexible, c'est-à-dire souple. Par «souple», il faut comprendre un article pouvant être comprimé ou pouvant fléchir sans se rompre, capable de s'adapter aux reliefs du corps humain. Un article souple réalisé sous la forme d'une nappe fibreuse peut dans certains exemples de réalisation être replié sur lui-même au moins une fois sans se casser en deux morceaux.

Cet article est généralement à usage unique.

Par ailleurs, l'article est généralement sec au toucher avant l'utilisation.

Après sa fabrication, l'article peut être par exemple conditionné en vrac dans une boîte ou dans un emballage individuel. Le cas échéant, les articles sont conditionnés en chapelet. Les articles peuvent encore être repliés sur eux-mêmes et intercalés, de telle sorte que le retrait d'un article amène le suivant dans une configuration facilitant sa préhension.

Ainsi, l'invention a encore pour objet, selon un autre de ses aspects, un ensemble comportant :
- un emballage,
- au moins un article tel que défini plus haut.

Dans le cas d'une composition colorée, l'article peut être conditionné dans un emballage comportant, le cas échéant, un témoin coloré représentatif de la couleur de la composition obtenue après dissolution de l'article, ceci afin de renseigner le consommateur avant l'achat.

L'invention offre ainsi de nouvelles possibilités pour le conditionnement et la formulation des produits cosmétiques à effet thermique, et leurs utilisations, par exemple comme produits d'hygiène ou de soin de la peau, des muqueuses, de la bouche ou des cheveux ou comme produits de maquillage.

Dans un exemple de mise en oeuvre de l'invention, l'article formé par le support et la composition contenant le composé à effet thermique, est mis en contact avec l'eau avant son utilisation. Le support est ainsi d'abord solubilisé juste avant que l'article soit appliqué sur le corps humain. Selon la quantité d'eau rajoutée à l'article pour solubiliser le support, on peut aisément ajuster la viscosité apparente de la composition obtenue.

Dans une autre variante de mise en oeuvre de l'invention, l'article formé par le support et la composition contenant le composé à effet thermique est mis au contact d'une région du corps humain, par exemple la peau ou les cheveux, avant sa solubilisation complète, voire avant d'être mouillé. Cela peut permettre par exemple, selon la quantité d'eau ajoutée, de modifier les propriétés en fonction du résultat souhaité. L'eau peut être versée ou pulvérisée sur l'article alors que celui-ci n'est pas au contact de la région du corps à traiter, ou bien la région du corps peut encore être mouillée, ou encore de l'eau peut être projetée ou versée sur le support alors que l'article est au contact de la région à traiter.

Au lieu d'utiliser de l'eau, on peut utiliser une composition aqueuse pour hydrater l'article, c'est-à-dire contenant au moins 50 % en poids d'eau par rapport au poids total de la composition, cette composition pouvant se présenter sous forme de lotion, de lait, de crème ou d'un gel, notamment un gel moussant.

### Support

Le support se présente sous forme d'une nappe comprenant des fibres hydrosolubles, c'est-à-dire des fibres solubles dans l'eau à une température inférieure ou égale à 30°C, de préférence solubles dans l'eau à une température inférieure ou égale à 20°C, c'est-à-dire ayant une température de dissolution dans l'eau allant de plus de 0°C à 30°C, de préférence de plus 0°C à 20°C, et par exemple de 5 °C à 30 °C, et mieux de 5 à 20 °C.

Le support peut être sensiblement non rétractable une fois mouillé.

De manière caractéristique, le support a une densité inférieure ou égale à 0,1 g/cm³, mieux allant de 0,01 g/cm³ à 0,1 g/cm³, ce qui permet d'avoir un support très aéré, qui, de ce fait, se dissout dans l'eau plus facilement.

Le support peut avoir toute forme appropriée pour l'utilisation visée, par exemple une forme rectangulaire, ronde ou ovale, et il a de préférence des dimensions permettant sa préhension entre deux doigts au moins. Ainsi, le support peut avoir par exemple une forme ovoïde d'environ 2 à 10 cm de long et d'environ 0,5 à 4 cm de large, ou une forme de disque d'environ 2 à 10 cm de diamètre, ou une forme de carré d'environ 5 à 15 cm de côté, ou une forme de rectangle d'une longueur d'environ 5 à 15 cm, étant entendu qu'il peut avoir toute autre forme et dimension appropriées pour l'utilisation recherchée.

Le support peut former par exemple un coussinet, un masque, un patch, une charlotte, un doigt de gant ou un gant, une nappe à découper, une lingette, un disque, un oval ou un rectangle. En outre, le support peut présenter une forme qui dépend de la région du corps à traiter.

Le support peut présenter une forme aplatie ou une forme non aplatie, présentant par exemple l'aspect d'un bloc formé d'un amas globulaire de fibres hydrosolubles compactées, incorporant une composition contenant un composé à effet thermique.

Les fibres du support sont généralement enchevêtrées pour former la nappe de fibres. Comme indiqué plus haut, on entend par « nappe comprenant des fibres solubles dans l'eau », une nappe pouvant être entièrement constituée de fibres solubles dans l'eau ou une nappe pouvant comporter à la fois des fibres solubles dans l'eau et des fibres insolubles dans l'eau, les fibres solubles devant être en plus grande quantité que les fibres insolubles. La nappe de fibres doit comporter au moins 60 % en poids de fibres solubles, de préférence au moins 70 % et mieux au moins 80 % en poids par rapport au poids total des fibres. Elle peut ainsi comporter, par exemple, plus de 95 % en poids, voire plus de 99 % en poids et même 100 % en poids de fibres hydrosolubles par rapport au poids total des fibres du support. Ainsi, le support peut être constitué entièrement de nappes de fibres solubles ou il peut être constituée de nappes comportant un mélange de fibres solubles et de fibres insolubles, les fibres insolubles étant selon la définition de la présente invention, des fibres qui ne sont pas solubles dans l'eau à une température inférieure ou égale à 30°C. Le fait d'avoir des fibres insolubles peut permettre d'avoir un produit à effet thermique qui soit en même temps un produit de gommage doux (scrub), les fibres insolubles constituant le composé exfoliant.

Ainsi, le support peut être formé de deux nappes constituées de fibres solubles dans l'eau, ou encore d'une nappe constituée de fibres solubles dans l'eau et d'une nappe comprenant à la fois de fibres solubles et de fibres insolubles, ou bien aussi d'une nappe constituée de fibres solubles dans l'eau et d'une nappe constituée de fibres insolubles dans l'eau, ou bien de deux nappes comprenant à la fois de fibres solubles et de fibres insolubles. Il peut y avoir aussi plus de deux nappes.

Selon un mode préféré de réalisation de l'invention, le support est dépourvu de fibres insolubles dans l'eau et il est composé uniquement de fibres solubles de l'eau, de sorte qu'il soit entièrement soluble dans l'eau.

Les fibres solubles peuvent être en tout matériau soluble susceptible d'étre filé en fibres. De préférence, les fibres solubles dans l'eau sont réalisées avec de l'alcool polyvinylique (PVA) selon un procédé qui leur confère la solubilité recherchée, le PVA pouvant avoir plusieurs grades de polymérisation.

Des fibres de PVA solubles dans l'eau à une température inférieure ou égale à 30°C sont commercialisées par la société japonaise KURARAY sous la dénomination commerciale KURALON K-II WN2. Le procédé de fabrication de ces fibres comporte la préparation d'une solution à filer par dissolution d'un polymère à base de PVA soluble dans l'eau, dans un premier solvant organique, le filage de la solution dans un second solvant organique pour obtenir des filaments solidifiés et l'étirage humide des filaments dont on enlève le premier solvant puis que l'on sèche et que l'on soumet à un traitement thermique. La section de ces fibres peut être sensiblement circulaire. Ces fibres ont une résistance à la traction d'au moins 2,7 g/dtex (3 g/d). La demande EP-A-0 636 716 décrit de telles fibres hydrosolubles à base de PVA et leur procédé de fabrication.

L'invention n'est pas limitée à l'emploi de PVA, et on peut utiliser aussi des fibres réalisées dans d'autres matériaux hydrosolubles sous réserve que ces matériaux se dissolvent dans de l'eau ayant la température recherchée, par exemple des fibres de polysaccharides commercialisées sous la dénomination LYSORB par la société LYSAC TECHNOLOGIES, INC ou des fibres à base de polymères polyholosides comme le glucomannane ou l'amidon.

La nappe de fibres peut comporter, le cas échéant, un mélange de différentes fibres solubles dans l'eau à des températures différentes (jusqu'à 30°C).

Les fibres peuvent être composites, et elles peuvent comporter par exemple un coeur et une gaine n'ayant pas la même nature, par exemple formés de différents grades de PVA.

Quand la nappe de fibres contient des fibres insolubles, celles-ci peuvent être en toute matière habituellement utilisée comme fibres insolubles ; ce peut être par exemple des fibres de soie, de coton, de laine, de lin, de cellulose extraites notamment du bois, des légumes ou des algues, de polyamide (Nylon^{®}), d'acide polylactique, de cellulose modifiée (rayonne, viscose, acétate notamment d'acétate de rayonne), de poly-p-phénylène téréphtalamide notamment de Kevlar^{®}, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon^{®}, de collagène insoluble, de polyesters, de polychlorure de vinyle ou de vinylidène, d'alcool polyvinylique, de polyacrylonitrile, de chitosane, de polyuréthane, de polyéthylène téréphtalate, de fibres formées d'un mélange des composés mentionnés ci-dessus, comme des fibres de polyamide/polyester ou de viscose/polyester. Les non tissés sont décrits de façon générale dans RIEDEL «Nonwoven Bonding Methods & Materials», Nonwoven World (1987), incorporé ici par référence.

Dans un exemple particulier de réalisation de l'invention, la nappe du support est un non-tissé, comportant des fibres hydrosolubles, seules ou en mélange avec des fibres insolubles comme indiqué plus haut, avec au plus de 40% en poids de fibres insolubles par rapport au poids total des fibres constituant la nappe. De préférence, le non-tissé est constitué de fibres hydrosolubles, c'est-à-dire qu'il ne contient pas de fibres insolubles.

Quand le support ne comporte qu'une seule nappe de fibres, la composition contenant le composé à effet thermique peut être déposée sur les deux faces du support ou sur une seule face, l'autre face du support pouvant alors être utilisée par exemple pour la préhension de l'article.

Quand le support selon la présente invention comporte deux nappes, il peut s'agir notamment de deux nappes de non tissé, tous les modes de réalisation décrits ci-dessous pouvant être utilisés, les nappes pouvant contenir ou non des fibres insolubles, et même une des nappes pouvant être constituée uniquement de fibres insolubles, du moment que l'autre nappe contient des fibres solubles.

Selon un mode particulier de réalisation de l'invention, chacune des nappes est un non-tissé constitué de fibres solubles à une température inférieure ou égale à 30°C, c'est-à-dire que les nappes ne comportent que des fibres hydrosolubles.

Selon un autre mode de réalisation, une des nappes est entièrement soluble dans l'eau et est un non-tissé constitué de fibres solubles à une température inférieure ou égale à 30°C, et l'autre nappe est insoluble et est est un non-tissé constitué de fibres insolubles.

Selon encore un autre mode de réalisation, le support comporte deux nappes contenant des fibres solubles ou partiellement solubles avec au plus 40 % de fibres insolubles, et en outre une nappe constituée de fibres insolubles, constituant un substrat insoluble. Ainsi, le support peut comporter au moins une couche d'un substrat insoluble dans l'eau, c'est-à-dire ne comportant que des fibres insolubles. Dans un exemple particulier de ce mode de réalisation, le support comprend une nappe soluble d'un non-tissé constitué de fibres solubles dans l'eau à une température inférieure ou égale à 30°C, et une nappe insoluble d'un non-tissé constitué de fibres insolubles dans l'eau.

Une structure multicouche avec au moins une couche formée d'un substrat insoluble dans l'eau peut par exemple être utile pour réaliser un article comportant un support en forme de doigt de gant. La couche formée de fibres hydrosolubles est située à l'extérieur de l'article, étant destinée à se solubiliser lors de l'utilisation, après avoir été mouillée ou en venant au contact d'une région mouillée du corps.

Pour fabriquer les nappes en non-tissé, quelles soient solubles ou insolubles, toutes les techniques appropriées de constitution d'un non-tissé à partir de fibres peuvent être utilisées. Par exemple, les fibres peuvent être formées par extrusion et déposées sur un convoyeur pour former une nappe de fibres qui est ensuite consolidée par une technique classique de liage de fibres, telle que par exemple l'aiguilletage, le liage à chaud, le calandrage ou le liage par jets d'air chaud (en anglais *air through bonding*), technique dans laquelle la nappe passe dans un tunnel où est insufflé de l'air chaud. Cette dernière technique est avantageusement utilisée lorsque la nappe est constituée de fibres bicomposant, par exemple des fibres comprenant au moins deux grades d'alcool polyvinylique (PVA), dont les points de fusion ou de ramollissement sont différents, ces fibres étant par exemple co-extrudées de manière à ce que la fibre soit constituée d'au moins un premier grade localisé au coeur de la fibre et d'au moins un deuxième grade localisé en périphérie de la fibre, sous la forme d'une gaine. Le liage des fibres peut être plus facile lorsque la gaine présente un point de fusion plus faible que le coeur.

La nappe de fibres peut encore être formée par cardage de fibres découpées à une longueur de 10 à 50 mm, puis dépôt des fibres sur un convoyeur où la nappe peut ensuite être consolidée par une technique de liage telle que décrite ci-dessus.

Lorsque le support comporte plusieurs couches, que celles-ci soient toutes réalisées avec des fibres hydrosolubles ou non, les différentes couches peuvent être assemblées de multiples manières, par exemple par soudage, collage ou couture, et ces couches peuvent constituer le cas échéant une ou plusieurs cavités contenant une ou plusieurs compositions cosmétiques ou dermatologiques ou plusieurs composants d'une même composition cosmétique à mélanger extemporanément. Lors d'un assemblage par couture, un fil lui-même hydrosoluble peut être utilisé, le cas échéant.

Quand le support comporte plusieurs nappes de non tissé, celles-ci peuvent être assemblées notamment par thermosoudage à leur périphérie de manière à constituer un coussinet capable de retenir dans une cavité intérieure, une composition contenant le composé à effet thermique.

Selon un autre aspect de l'invention, le support est dépourvu d'adhésif, notamment d'adhésif sensible à la pression.

La composition contenant au moins un composé à effet thermique représente entre 10 et 1000 % en poids par rapport au poids du support, et de préférence entre 10 et 500 % en poids par rapport au poids du support en entendant ici par «poids du support», le poids du support seul, sans le poids de la composition contenant le composé à effet thermique. Si la composition ne contient que le composé à effet thermique, c'est celui-ci qui peut représenter entre 10 et 1000 % en poids par rapport au poids du support, et de préférence entre 10 et 500 % en poids par rapport au poids du support.

### Composés à effet thermique

On entend dans la présente demande par « composés à effet thermique » des composés provoquant un changement de température quand ils sont mis en contact avec de l'eau, ce contact pouvant se produire par addition d'eau au moment de l'application sur la matière kératinique telle que la peau, ou par simple application sur la matière kératinique, l'eau nécessaire à la réaction exothermique ou endothermique étant apportée directement par la matière kératinique elle-même, préalablement mouillée ou non, ou étant apportée après application sur la matière kératinique. Ces composés sont notamment des composés endothermiques ou exothermiques.

L'article de l'invention contient un ou plusieurs composés susceptibles de provoquer un changement de température, c'est-à-dire de dégager de la chaleur ou du froid, au contact de l'eau. La quantité en composé(s) à effet thermique doit être telle que l'utilisateur ressente effectivement un effet chaud ou un effet froid lors de l'utilisation de l'article. Le ou les composés à effet thermique peuvent représenter 100 % de la composition portée par le support. Ils peuvent être présents en une quantité allant par exemple de 10 à 100 % du poids total de la composition portée par le support, et de préférence de 20 à 100 % en poids par rapport au poids total de la composition portée par le support. On entend dans la demande par « % en poids par rapport au poids total de la composition » le pourcentage en poids par rapport au poids total de la composition portée par le support (et non par rapport au poids de l'article comprenant support et composition).

Comme composés donnant des effets chauds, composés exothermiques, on peut citer notamment les zéolithes (activées ou non activées), les sels inorganiques exothermiques, les polyols ayant au moins 2 groupes hydroxyle et au moins 3 atomes de carbone, les éthers d'alcool vanillylique, le gingerol ; la capsaïcine et ses dérives ; l'eugénol ; l'huile de cannelle ; l'alcool benzylique, les systèmes redox, et leurs mélanges.

A titre de zéolites (silicoaluminates), on peut citer par exemple les zéolites A, les zéolites X comme celles commercialisées par les sociétés Fluka et Union

Carbide, les zéolites MAP comme décrit dans le document EP-A-384070, les zéolites A activées telles que décrites dans le documents EP-A-187912. Les cations présents dans les zéolites utilisées comprennent notamment Na, K, Ca, Zn, Mg, Li, Cu et leurs combinaisons.

Comme sels inorganiques exothermiques, on peut citer plus particulièrement le chlorure de calcium, le chlorure de magnésium, et les mélanges les contenant.

A titre de polyols ayant au moins 2 groupes hydroxyle et au moins 3 atomes de carbone, on peut citer notamment la glycérine, la diglycérine, le propylène glycol, le butylène glycol, l'hexylène glycol, le polyéthylène glycol et les polyéthylène glycols de poids moléculaire inférieur à 600 comme le PEG-8, les sucres tels que le sorbitol, et leurs mélanges.

Comme éthers d'alcool vanillylique, on peut citer par exemple le n-butyl éther d'alcool vanillylique, le n-propyl éther d'alcool vanillylique, l'isopropyl éther d'alcool vanillylique, l'isobutyl éther d'alcool vanillylique, l'isoamyl éther d'alcool vanillylique, le n-hexyl éther d'alcool vanillylique le methyl éther d'alcool vanillylique, l'éthyl éther d'alcool vanillylique.

Les systèmes redox peuvent être notamment basés sur l'association entre une poudre de fer et un catalyseur à haute surface spécifique, de type alumine, aluminosilicate, silice ou charbon, le rapport en poids de la poudre de fer sur la catalyseur pouvant aller par exemple de 1000 :1 à 1 :1000. Un tel système est décrit par exemple dans le document WO-A-01 /12133.

Comme composés donnant des effets froids, composés endothermiques, on peut citer par les sels inorganiques endothermiques comme le chlorure de potassium ; la menthe et ses dérivés, ou des composés azotés tels que l'urée.

Comme dérivés de la menthe, on peut citer par exemple le menthol, l'huile de menthe poivrée (peppermint), le wintergreen, la menthone, le menthyl lactate, la menthe verte, l'huile de menthe ; les dérivés de menthane comme les menthane carboxamides N-substitués, le 3- (I-menthoxy)-propane-1,2-diol, le p-menthane-3,8,-diol, le menthyl succinate et ses sels alcalinoterreux, et leurs mélanges.

Selon un mode préféré de réalisation de l'invention, les composés à effet thermique se présentent sous forme pulvérulente ou pâteuse, de préférence sous forme pulvérulente. Toutefois, il est possible d'utiliser des composés liquides qui peuvent être par exemple absorbés sur des poudres ou encapsulés avant d'être mis sur le support ou mélangé avec d'autres composés dans la composition mise sur le support.

Comme indiqué ci-dessus, les composés à effet thermique utilisés selon l'invention peuvent être mis tels quels sur le support ou être incorporés dans une composition mise sur le support.

### Compositions

Les compositions contenant le ou les composés à effet thermique sont des compositions anhydres. Elles sont de préférence sous forme pulvérulente ou pâteuse, et plus préférentiellement sous forme pulvérulente. Ce sont des compositions adaptées à une application topique, notamment des compositions cosmétiques ou dermatologiques.

Ainsi, les compositions utilisables dans l'invention peuvent être par exemple :
- des émulsions lyophilisées ou atomisées, telles que celles décrites dans le document FR-A-2,727,312 ou celles à base d'amidon modifié, décrites dans le document EP-A-0 938 892. Ces émulsions sont obtenues par lyophilisation ou atomisation d'une émulsion H/E contenant une phase pulvérulente,
- des compositions moussantes sous forme de poudres, contenant des tensioactifs pulvérulents, comme celles à base d'amidon, décrites dans le document EP-A-0 925 777,
- des compositions pulvérulentes exemptes d'huile, contenant principalement des agents gélifiants (polymères, argiles) et/ou des tensioactifs,
- des compositions formées par simple mélange des constituants, ceux-ci étant de préférence sous forme de poudres.

La composition peut ne contenir que le composé à effet thermique qui représente alors 100 % du poids de la composition.

Selon les constituants des compositions utilisées, l'article se transforme en lait, en crème, en mousse, en gel, ou en lotion après humidification.

La composition peut éventuellement contenir une certaine quantité d'eau au moment de son imprégnation sur le support. Toutefois, de manière à éviter sa solubilisation prématurée, l'eau introduite sur le support lors de son imprégnation doit être éliminée par les moyens classiquement utilisés pour la déshydratation des compositions contenant de l'eau, comme par exemple le chauffage. Cependant, la composition peut contenir une certaine quantité d'eau qui est généralement de l'eau liée qui peut provenir notamment des matières premières hygroscopiques qui contiennent de l'eau, telles que les amidons La quantité d'eau finale dans la composition présente sur l'article est d'au maximum 10 % en poids et de préférence au maximum 5 % en poids par rapport au poids total de la composition.

Lorsque la composition doit être déposée sur le support par l'utilisateur lui-même, la composition et le support peuvent être proposés ensemble, sous la forme d'un kit, par exemple. La composition est par exemple livrée en une quantité suffisante pour permettre d'en distribuer une pluralité de doses sur un ensemble de supports destinés à être utilisés successivement.

### Autres ingrédients

Les autres ingrédients de la composition contenant le composé à effet thermique dépendent de l'utilisation finale de l'article. Comme autres ingrédients, on peut citer par exemple les tensioactifs moussants, les polymères, les composés lipophiles, les exfoliants, ainsi que les actifs et les additifs habituellement utilisés dans les domaines concernés. Si nécessaire, les additifs peuvent être encapsulés ou adsorbés sur des poudres.

Selon un mode particulier de réalisation de l'invention, la composition portée par le support contient en outre au moins un composé choisi parmi les tensioactifs moussants, les polymères, les composés lipophiles, les exfoliants, les actifs, et leurs mélanges.

### Tensioactifs moussants

Quand l'article selon l'invention doit conduire à une composition moussante ou de nettoyage, la composition contenant le composé à effet thermique contient en outre au moins un tensioactif moussant, de préférence sous forme pulvérulente (poudre). Comme tensioactifs moussants, on peut utiliser tous ceux habituellement utilisés dans le domaine cosmétique, ces tensioactifs pouvant être anioniques, non ioniques, cationiques, amphotères ou zwitterioniques.

La quantité de tensioactif(s) moussant(s) peut aller par exemple de 2 à 80 % en poids, de préférence de 10 à 70 % en poids par rapport au poids total de la composition.

Comme tensioactifs anioniques moussants, on peut citer par exemple les sels d'acide gras qui constituent les savons et qui sont dérivés d'un acide gras ayant une chaîne alkyle comportant de 6 à 22 atomes de carbone, de préférence de 8 à 18 atomes de carbone, notamment de sels obtenus par neutralisation d'un acide gras par une base organique ou minérale telle que la potasse, la soude, la triéthanolamine, la N-methylglucamine, la lysine et l'arginine. Comme sels d'acides gras (savons), on peut citer notamment les sels alcalins et par exemple les sels de potassium ou de sodium des acides laurique, myristique, palmitique, stéarique (laurate, myristate, palmitate et stéarate de potassium ou de sodium).

On peut aussi citer comme tensioactifs anioniques, les alkylsulfates et alkylethersulfates ; les sulfonates,; les sels alcalins de N-acylaminoacides tels que les sarcosinates, les alaninates, les glutamates, les aspartates, les glycinates ; et leurs mélanges.

Comme tensioactifs non ioniques, on peut citer par exemple les esters de sucre, les éthers de sucre comme les alkyl polyglucosides (APG), les condensats d'oxydes d'alkylène et d'alkyl phénols, les éthers d'alcool gras et de polyols, et leurs mélanges.

Comme tensioactifs amphotères ou zwitterioniques, on peut citer les bétaïnes et leurs dérivés, les sultaïnes et leurs dérivés, les dérivés d'imidazolinium, et leurs mélanges.

Les tensioactifs préférés sont ceux en poudre, tels que par exemple le lauryl sulfate de sodium comme le produit commercialisé sous la dénomination Empicol LZ D par la société Allbright & Wilson ou sous la dénomination Tensopol USP97 par la société Tensachem ; la cocamidopropylbetaine comme le produit commercialisé sous la dénomination Tegobetain CK D par la société Degussa ; le lauroyl glutamate de sodium comme le produit commercialisé sous la dénomination Amisoft LS 11 par la société Ajinomoto ; le myristoyl glutamate monosodique comme le produit commercialisé sous la dénomination Acylglutamate MS 11 par la société Ajinomoto ; le mélange de laureth sulfate de sodium et de silice, commercialisé sous la dénomination Texapon KE 2713 par la société Cognis ; le disodium cocamido MEA-sulfosuccinate comme le produit commercialisé sous la dénomination Mackanate CM 100 par la société Mac Intyre ; le methyl cocoyl taurate de sodium, comme le produit commercialisé sous la dénomination Tauranol WSP par la société Finetex ; le decyl d-galactoside uronate de sodium comme le produit commercialisé sous la dénomination Decyl d-galactoside uronate de sodium par la société Ard-Soliance ; le lauroyl methyl beta-alanine (forme acide) commercialisé sous la dénomination LMA-H par la société Mitsui Toatsu ; la n-lauroyl-n-hydroxyethyl-beta-alanine commercialisée sous la dénomination LHEA par la société Mitsui Toatsu ; le cocoyl glycinate de sodium commercialisé sous la dénomination Amilite GCS-11(F) par la société Ajinomoto ; le cocoyl isethionate de sodium comme le produit commercialisé sous la dénomination Jordapon CI P par la société BASF ; le lauryl sulfoacétate de sodium, comme le produit commercialisé sous la dénomination Lathanol LAL poudre par la société Stepan ; le myristate de potassium comme le produit commercialisé sous la dénomination Myristate de potassium (DUB MK) par la société Stearinerie Dubois ; le laurate de potassium comme le produit commercialisé sous la dénomination Laurate de potassium (DUB LK) par la société Stearinerie Dubois, et le laurate de sucrose comme le produit commercialisé sous la dénomination Grilloten LSE 87 par la société Degussa.

Selon un mode préféré de réalisation de l'invention, quand l'article selon l'invention doit conduire à une composition moussante ou de nettoyage, la composition contenant le composé à effet thermique contient au moins un tensioactif moussant anionique.

### Polymères

La composition portée par le support peut contenir aussi un ou plusieurs polymères, notamment des polymères hydrosolubles. A titre d'exemple de polymères hydrosolubles utilisables dans l'invention, on peut citer les gommes de guar, de xanthane, de carraghénane, de cellulose, de sclerotium, les dérivés de ces gommes, les hydroxyalkylcelluloses, la carboxyméthylcellulose, les polyacrylamides et les copolymères d'acrylamides et notamment les homopolymères et copolymères d'acide 2-acrylamido 2-méthylpropane sulfonique tels que ceux commercialisés sous les dénominations Hostacerin AMPS et Aristoflex par la société Clariant, la gélatine, l'agar-agar, les polymères carboxyvinyliques tels que les produits commercialisés sous les dénominations Carbopol par la société Noveon (nom INCI: carbomer), les polymères carboxyvinyliques modifiés et notamment les copolymères acrylate/C₁₀-C₃₀-alkylacrylate tels que les produits commercialisés sous les dénominations Pemulen TR1 ou TR2 ou CARBOPOL 1382 par la société Noveon (nom INCI : Acrylates/C10-30 Alkyl acrylate Crosspolymer), la montmorillonite et le silicate de magnésium et aluminium.

Lorsqu'ils sont présents, la quantité de polymère(s) dans la composition de l'invention peut aller par exemple de 0,1 à 80 % et de préférence de 0,5 à 70 % du poids total de la composition portée par le support

### Composés lipophiles

La composition selon l'invention peut contenir aussi une ou plusieurs composés lipophiles, corps gras et notamment huiles, ou actif huileux. La quantité de composé lipophile peut aller par exemple de 1 à 80 % en poids par rapport au poids total de la composition portée par le support.

On peut utiliser toute sorte d'huiles et de corps gras bien connus de l'homme du métier, comme par exemple les huiles d'origine végétale (par exemple huiles de jojoba, avocat, sésame, tournesol, maïs, soja, carthame, pépins de raisin), les huiles minérales (par exemple vaseline, isoparaffines éventuellement hydrogénées), les huiles de synthèse (par exemple myristate d'isopropyle, octanoate de cétéaryle, polyisobutylène, palmitate ou myristate d'éthyl-hexyle, alkyl-benzoates), les huiles de silicone volatiles ou non volatiles, et les huiles fluorées ou fluorosiliconées, ainsi que les mélanges de ces huiles.

Comme autres corps gras, on peut citer les alcools gras comme l'alcool stéarylique, l'alcool cétylique et leur mélange (alcool cétéarylique), les acides gras, les gommes, par exemple les gommes de silicone comme le mélange PDMS à groupements alpha oméga hydroxylés / PDMS 5 est (12/88) vendu sous la dénomination DC 1503 par la société Dow Corning, et les gélifiants lipophiles tels que la bentone.

### Exfoliants

La composition peut contenir aussi des exfoliants, notamment pour constituer une composition de gommage ou un scrub pour le visage ou le corps. Comme exfoliants, on peut citer par exemple des particules exfoliantes ou gommantes d'origines minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges. Par ailleurs, comme indiqué plus haut, l'exfoliant peut être constitué de fibres insolubles incluses dans la nappe de fibres du support.

Les particules exfoliantes peuvent être présentes en une quantité allant par exemple de 0,5 à 40 % en poids, de préférence de 1 à 20 % en poids et mieux de 1 à 10 % en poids par rapport au poids total de la composition. Quand la composition contient des particules exfoliantes, l'article obtenu peut être utilisé notamment pour le gommage de la peau du visage ou du corps.

### Actifs

Les actifs peuvent être choisis notamment parmi les agents kératolytiques, les hydratants, les apaisants et les antimicrobiens. Si nécessaire, les actifs peuvent être encapsulés ou adsorbés sur des poudres.

Comme hydratants, on peut citer les polyols tels que la glycérine ; les composés agissant sur la fonction barrière, en vue de maintenir l'hydratation du stratum corneum, ou les composés occlusifs, en particulier les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ; les composés augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ; et leurs mélanges.

Comme agents kératolytiques, on peut citer les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique, et leurs mélanges.

Comme agents apaisants utilisables dans la composition selon l'invention, on peut citer par exemple les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, les extraits de plantes telles que *Paeonia suffruticosa* et / ou *lactiflora, Laminaria saccharina, Boswellia serrata, Centipeda cunnighami, Helianthus annuus, Linum usitatissimum, Cola nitida, Epilobium Angustifolium, Aloe vera, Bacopa monieri,* les sels de l'acide salicylique et en particulier le salicylate de zinc, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier muscat, de cassis, d'ecchium, ou de poisson, des extraits de plancton, la capryloyl glycine, le Seppicalm VG (sodium palmitoylproline et nymphea alba) de Seppic, les tocotrienols, le piperonal, un extrait de clou de girofle, les phytostérols, la cortisone, l'hydrocortisone, l'indométhacine et la beta méthasone.

Comme antimicrobiens, on peut citer par exemple le 2,4,4'-trichloro-2'-hydroxy diphényl éther (ou triclosan), le 3,4,4'-trichlorocarbanilide (ou triclocarban) le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO-A-93/18743, le farnesol, les phytosphingosines et leurs mélanges.

Comme vitamines, on peut utiliser les vitamines ou provitamines hydrosolubles ou liposolubles, comme par exemple les vitamines A (rétinol), C (acide ascorbique), B3 ou PP (niacinamide), B5 (panthénol), B6 ou pyridoxine, E (tocophérol), K1, le bêta-carotène, et les dérivés de ces vitamines et notamment leurs esters, et leurs mélanges.

### Additifs

La composition de l'invention peut contenir un ou plusieurs additifs, notamment ceux qui sont anhydres ou sous forme solide (poudre), choisis parmi ceux généralement utilisés dans les domaines cosmétique et dermatologique, tels que, par exemple, les séquestrants, les parfums, les antioxydants, les conservateurs, les matières colorantes (comme les pigments et les colorants hydrophiles) et les charges minérales et/ou les charges organiques telles que l'amidon modifié comme celui commercialisé sous la dénomination Dry Flo par la société National Starch. Si nécessaire, les additifs peuvent être encapsulés ou adsorbés sur des poudres.

Bien entendu, l'homme de l'art veillera à choisir ce ou ces éventuels adjuvants et additifs et/ou leurs quantités de manière telle que les propriétés avantageuses attachées intrinsèquement à la composition conforme à l'invention ne soient pas, ou substantiellement pas, altérées par la ou les adjonctions envisagées.

Dans le domaine du soin et/ou du nettoyage de la peau, l'article selon l'invention peut être utile dans diverses applications, notamment pour le nettoyage et le démaquillage de la peau, le traitement des signes de l'âge, le traitement des peaux grasses, l'hydratation, la photoprotection, le traitement des peaux sensibles ou sensibilisées pour les apaiser en cas d'irritation. Ainsi, il peut constituer par exemple un produit de nettoyage ou de démaquillage de la peau, un produit exfoliant, un produit de soin de la peau notamment un produit hydratant, un patch à rincer, un produit de maquillage. Il peut aussi être utilisé dans le domaine capillaire comme un produit capillaire.

L'article selon l'invention peut être présenté dans différents buts d'application :
- comme applicateur 2-en-1, l'article servant à appliquer un produit sans être mouillé et étant mouillé seulement après application dudit produit. Par exemple, on peut l'utiliser comme applicateur d'un démaquillant sensiblement anhydre, l'effet thermique étant obtenu par mouillage de l'article après application du démaquillant, ou comme applicateur d'un produit moussant tonifiant en réhydratant l'article ensuite dans les mains ;
- comme produit unidose à réhydrater, par exemple comme crème exfoliante chauffante en humidifiant et en solubilisant l'article dans les mains ;
- comme patch à rincer, un patch étant destiné à être appliqué sur une zone limitée : par exemple, comme patch/gel rafraîchissant pour peaux grasses, en humidifiant la zone à traiter et en y appliquant le patch qui se transforme en gel sur la peau, puis en l'éliminant par simple rinçage ;
- comme article 2-en-1 : comme patch à rincer pour le traitement spécifique d'une zone restreinte (application du patch sur zone humidifiée puis rinçage après un temps de pose) ou comme produit unidose à appliquer sur une plus large zone en ayant préalablement humidifié le produit.

Quand l'article est un produit de nettoyage ou démaquillage, un patch à rincer ou un shampoing, il faut rincer après application du produit et après éventuellement un temps de pose.

Les exemples qui suivent servent à illustrer l'invention sans toutefois présenter un caractère limitatif. Les quantités indiquées sont en % en poids sauf mention contraire, et elles correspondent sauf mention contraire à la quantité de matière première et non à la quantité de matière active. Les noms des composés utilisés sont indiqués en nom INCI, en nom chimique ou en nom commercial.

### EXEMPLES

L'article utilisé dans les exemples a été réalisé avec un support en fibres Kuralon K-II WN2 à base de PVA, fibres qui sont solubles à une température inférieure ou égale à 20°C. Il a été obtenu en thermosoudant à leur périphérie deux couches de grammage 80 g/m². L'article se présentait sous la forme d'un disque de 3 cm de diamètre, comportant une cavité dans laquelle a été introduite la composition contenant le composé à effet thermique.

Pour les exemples 1 et 3, l'article contenait 0,3 g des compositions décrites.

Pour les exemples 2 et 4, l'article contenait 0,5 g des compositions décrites.

Pour utiliser l'article obtenu dans ces exemples, on peut le disposer dans la paume de la main, le mouiller avec environ 2 à 4 ml d'eau et l'appliquer sur le visage, ou on peut le mettre directement sur la peau humidifiée. Puis on rince la peau.

| | Exemple 1 selon l'invention : Produit moussant tonifiant | Exemple 2 selon l'invention : Crème démaquillante exfoliante |
|---|---|---|
| Sodium Cocoyl Isethionate (1) | 40 | - |
| Potassium myristate | 40 | - |
| Urée | 20 | - |
| Amidon modifié (2) | - | 17.5 |
| MgCl₂ | - | 20 |
| Poudre de polyéthylène (3) | - | 10 |
| Huile de vaseline | - | 52.5 |

| | | |
|---|---|---|
| (1) Jordapon CIP (BASF) (2) C* Flo 06205 (Cerestar) (3) Microthene MN 727 (Equistar) | | |

L'exemple 1 a été préparé en mélangeant les poudres, puis en introduisant le mélange dans la cavité du support, qui a été ensuite fermée par soudage.

L'exemple 2 a été préparé en réalisant une émulsion H/E en mélangeant les composés sauf MgCl₂, avec environ 70% d'eau, puis en éliminant l'eau par atomisation et en ajoutant MgCl₂. Le mélange pulvérulent obtenu a été introduit dans la cavité du support, qui a été ensuite fermée par soudage.

L'exemple 1 contient de l'urée qui a donné un effet froid après hydratation de l'article, tandis que l'exemple 2 contient du chlorure de magnésium qui a donné un effet chaud après hydratation de l'article.

| | Exemple 3 selon l'invention : Patch peaux grasses à rincer | Exemple 4 selon l'invention : Patch apaisant après-soleil |
|---|---|---|
| Polyvinyl alcool (1) | 40 | - |
| Carboxyméthyl cellulose (2) | - | 70 |
| Acide salicylique | 10 | - |
| Zéolite (3) | 20 | - |
| Kaolin | 30 | - |
| Actif apaisant (4) | - | 2 |
| urée | - | 28 |

| | | |
|---|---|---|
| (1) Celvol 540 PV alcohol (Celanese chemical) (2) Blanose 9M31 F (Hercules) (3) X-mol (Zeochem) (4) N acetyl-tyr-arg hexadecyl ester (Sederma) | | |

Les exemples 3 et 4 ont été préparés en mélangeant les poudres, puis en introduisant le mélange dans la cavité du support, qui a été ensuite fermée par soudage.

L'exemple 3 contient de la zéolithe qui a donné un effet chaud après hydratation tandis que l'exemple 4 contient de l'urée qui a donné un effet froid après hydratation.

## Revendications

1. Article cosmétique ou dermatologique comportant :
- un support sous forme d'au moins une nappe comprenant des fibres solubles dans l'eau à une température inférieure ou égale à 30°C, ledit support présentant une densité inférieure ou égale à 0,1 g/cm³, et
- une composition portée par le support, contenant au moins un composé à effet thermique, **caractérisé en ce que** le support comporte au moins deux nappes définissant entre elles une cavité, l'une au moins des nappes comprenant des fibres solubles dans l'eau à une température inférieure ou égale à 30°C, et la cavité contient la composition contenant au moins un composé à effet thermique.

2. Article selon la revendication 1, **caractérisé en ce que** les fibres solubles dans l'eau à une température inférieure ou égale à 30°C sont réalisées avec de l'alcool polyvinylique.

3. Article selon la revendication précédente, **caractérisé en ce que** les deux nappes de fibres sont des non-tissés.

4. Article selon la revendication précédente, **caractérisé en ce que** l'une des nappes est un non-tissé constitué de fibres solubles dans l'eau à une température inférieure ou égale à 30°C, et l'autre nappe est un non-tissé constitué de fibres insolubles dans l'eau.

5. Article selon l'une quelconque des revendications précédentes, **caractérisé en ce que** les deux nappes sont assemblées à leur périphérie.

6. Article selon la revendication précédente, **caractérisé en ce que** les nappes sont thermosoudées.

7. Article selon l'une quelconque des revendications 1 à 3, 5 ou 6, **caractérisé en ce que** le support est entièrement soluble dans l'eau.

8. Article selon l'une quelconque des revendications précédentes, **caractérisé en ce que** la quantité de composé(s) à effet thermique va de 10 à 100 % en poids par rapport au poids total de la composition.

9. Article selon l'une quelconque des revendications précédentes, **caractérisé en ce que** le composé à effet thermique est un composé exothermique choisi parmi les zéolithes, les sels inorganiques exothermiques, les polyols ayant au moins 2 groupes hydroxyle et au moins 3 atomes de carbone, les éthers d'alcool vanillylique, le gingero, la capsaïcine et ses dérives, l'eugénol, l'huile de cannelle, l'alcool benzylique, les systèmes redox, et leurs mélanges.

10. Article selon l'une quelconque des revendications précédentes, **caractérisé en ce que** le composé à effet thermique est un composé endothermique choisi parmi les sels inorganiques endothermiques, la menthe et ses dérivés, l'urée, et leurs mélanges.

11. Article selon l'une quelconque des revendications précédentes, **caractérisé en ce que** le composé à effet thermique ou la composition contenant le composé à effet thermique sont sous forme pulvérulente ou pâteuse.

12. Article selon l'une quelconque des revendications précédentes, **caractérisé en ce que** la composition portée par le support représente entre 10 et 1000 % en poids par rapport au poids du support.

13. Article selon l'une quelconque des revendications précédentes, **caractérisé en ce que** la composition portée par le support contient en outre au moins un composé choisi parmi les tensioactifs moussants, les polymères, les composés lipophiles, les exfoliants, les actifs, et leurs mélanges.

14. Article selon l'une quelconque des revendications précédentes, **caractérisé en ce qu'**il constitue un produit de nettoyage ou de démaquillage de la peau, un produit exfoliant, un produit de soin de la peau, un patch à rincer, un produit capillaire, un produit de maquillage.

15. Article selon l'une quelconque des revendications précédentes, **caractérisé en ce que** le support forme un coussinet, un masque, un patch, une charlotte, un doigt de gant, un gant, une nappe à découper, une lingette, un disque, un ovale ou un rectangle.

16. Composition pour application topique, obtenue par la dissolution dans l'eau, d'un article selon l'une quelconque des revendications 1 à 15.

17. Procédé de traitement cosmétique d'une matière kératinique, comportant :
- la formation d'une composition cosmétique par dissolution dans l'eau, d'un support comportant au moins une nappe de fibres solubles dans l'eau à une température inférieure ou égale à 30°C, ledit support présentant une densité inférieure ou égale à 0,1 g/cm³, et portant au moins un composé à effet thermique, ledit support étant **caractérisé en ce qu'**il comporte au moins deux nappes définissant entre elles une cavité, l'une au moins des nappes comprenant des fibres solubles dans l'eau à une température inférieure ou égale à 30°C, et la cavité contient la composition contenant au moins un composé à effet thermique.
- l'application de la composition ainsi formée sur la matière kératinique.

18. Ensemble comportant :
- un emballage,
- au moins un article selon l'une quelconque des revendications 1 à 15.

## Claims

1. Cosmetic or dermatologic particle, comprising:
- a support in the form of at least one sheet comprising fibres that are soluble in water at a temperature less than or equal to 30°C, said support having a density less than or equal to 0.1 g/cm², and
- a composition carried by the support, containing at least one compound with thermal effect, **characterized in that** the support comprises at least two sheets defining a cavity between them, with aft least one of the sheets comprising fibres that are soluble in water at a temperature less than or equal to 30°C, and the cavity contains the composition containing at least one compound with thermal effect.

2. Article according to Claim 1, **characterized in that** the fibres that are soluble in water at a temperature less than or equal to 30°C are made with polyvinyl alcohol.

3. Article according to the preceding claim, **characterized in that** the two sheets of fibres are nonwovens.

4. Article according to the preceding claim, **characterized in that** one of the sheets is a nonwoven, constituted of fibres that are soluble in water at a temperature less than or equal to 30°C, and the other sheet is a nonwoven, constituted of water-insoluble fibres.

5. Particle according to any one of the preceding claims, **characterized in that** the two sheets are joined together at their periphery.

6. Article according to the preceding claim, **characterized in that** the sheets are heat-sealed.

7. Article according to any one of Claims 1 to 3, 5 or 6, **characterized in that** the support is completely soluble in water.

8. Article according to any one of the preceding claims, **characterized in that** the amount of compound(s) with thermal effect ranges from 10 to 100 wt.% relative to the total weight of the composition.

9. Article according to any one of the preceding claims, **characterized in that** the compound with thermal effect is an exothermic compound selected from the zeolites, exothermic inorganic salts, polyols having at least 2 hydroxyl groups and at least 3 carbon atoms, ethers of vanillyl alcohol, gingerol, capsaicin and its derivatives, eugenol, cinnamon coil, benzyl alcohol, redox systems, and mixtures thereof.

10. Article according to any one of the preceding claims, **characterized in that** the compound with thermal effect is an endothermic compound selected from the endothermic inorganic salts, mint and its derivatives, urea, and mixtures thereof.

11. Article according to any one of the preceding claims, **characterized in that** the compound with thermal effect or the composition containing the compound with thermal effect is in the form of powder or paste.

12. Article according to any one of the preceding claims, **characterized in that** the composition carried by the support represents between 10 and 1000 wt. % relative to the weight of the support.

13. Article according to any one of the preceding claims, **characterized in that** the composition carried by the support contains in addiction at least one compound selected from the foaming surfactants, polymer, lipophilic compounds, exfoliating agents, active agents, and mixtures thereof.

14. Article according to any one of the preceding claims, **characterized in that** it constitutes a skin cleansing or make-up removal product, an exfoliator, a skin care product, a rinsing patch, a hair care product, or a make-up product.

15. Article according to any one of the preceding claims, **characterized in that** the support forms a pad, a mask, a patch, a mobcap, a finger of a grove, a glove, a sheet for cutting out, a wipe, a disc, an oval or a rectangle.

16. Composition for tropical application, obtained by dissolving, in water, an particle according to any one of Claims 1 to 15.

17. Method of cosmetic treatment of a keratinous material, comprising:
- the formation of a cosmetic composition by dissolving, in water, a support comprising at least one sheet of fibres that are soluble in water at a temperature less than or equal to 30°C, said support having a density less than or equal to 0.1 g/cm₃, and bearing at least one compound with thermal effect, said support being **characterized in that** it comprises at least two sheets defining a cavity between them, with at least one of the sheets comprising fibres that are soluble in water at a temperature less than or equal to 30°C, and the cavity contains the composition containing at least one compound with thermal effect;
- application of the composition thus formed on the keratinous maternal.

18. Kit comprising:
- a packet,
- at least one article according to any one of Claims 1 to 15.

## Patentansprüche

1. Kosmetischer oder dermatologischer Artikel, umfassend:
- einen Träger in Form mindestens einer Lage, die bei einer Temperatur kleiner gleich 30°C wasserlösliche Fasern umfasst, wobei der Träger eine Dichte kleiner bleich 0,1 g/cm³ aufweist, und
- eine von dem Träger getragene Zusammensetzung, die mindestens eine Verbindung mit thermischem Effekt enthält, **dadurch gekennzeichnet, dass** der Träger mindestens zwei Lagen umfasst, die zusammen einen Hohlraum definieren, wobei mindestens eine der Lagen bei einer Temperatur kleiner gleich 30°C wasserlösliche Fasern umfasst, und der Holraum die mindestens eine Verbindung mit thermischem Effekt enthaltende Zusammensetzung enthält.

2. Artikel nach Anspruch 1, **dadurch gekennzeichnet, dass** die bei einer Temperatur kleiner gleich 30°C wasserlöslichem Fasern mit Polyvinylalkohol gefertigt sind.

3. Artikel nach dem vorhergehenden Anspruch, **dadurch gekennzeichnet, dass** es sich bei den beiden Faserlagen um Vliesstoffe handelt.

4. Artikel nach dem vorhergehenden Anspruch, **dadurch gekennzeichnet, dass** es sich bei der einen der Lagen um einen Vliesstoff aus bei einer Temperatur kleiner gleich 30°C wasserlöslichem Fasern handelt und es sich bei der anderen Lage um einen Vliesstoff aus wasserunlöslichen Fasern handelt.

5. Artikel nach einem der vorhergehenden Ansprüche, **dadurch gekennzeichnet, dass** die beiden Lagen an ihrem Rand zusammengefügt sind.

6. Artikel nach dem vorhergehenden Anspruch, **dadurch gekennzeichnet, dass** die Lagen warmverschweißt sind.

7. Artikel nach einem der Ansprüche 1 bis 3, 5 oder 6, **dadurch gekennzeichnet, dass** der Träger vollständig wasserlöslich ist.

8. Artikel nach einem der vorhergehenden Ansprüche, **dadurch gekennzeichnet, dass** die Menge der Verbindung bzw. Verbindungen mit thermischem Effekt 10 bis 100 Ges.-%, bezogen auf das Gesamtgewicht der Zusammensetzung, beträgt.

9. Artikel nach einem der vorhergehenden Ansprüche, **dadurch gekennzeichnet, dass** es sich bei der Verbindung mit thermischem Effekt um eine exotherme Verbindung, die unter Zeolithen, exothermen anorganischen Salzen, Polyolen mit mindestens 2 Hydroxylgruppen und mindestens 3 Kohlenstoffatomen, Ethern von Vanillylalkohol, Gingerol, Capsaicin und Derivaten davon, Eugenol, Zimtöl, Benzylalkohol, Redoxsysteme und Mischungen davon ausgewählt ist, handelt.

10. Artikel nach einem der vorhergehenden Ansprüche, **dadurch gekennzeichnet, dass** es sich bei der Verbindung mit thermischem Effekt um eine endotherme Verbindung, die unter endothermen anorganischen Salzen, Minze und Derivaten davon, Harnstoff und Mischungen davon ausgewählt ist, handelt.

11. Artikel nach einem der vorhergehenden Ansprüche, **dadurch gekennzeichnet, dass** die Verbindung mit thermischem Effekt bzw. die Zusammensetzung, die die Verbindung mit thermischem Effekt enthält, in Pulver- oder Pastenform vorlegt.

12. Artikel nach einem der vorhergehenden Ansprüche, **dadurch gekennzeichnet, dass** die von dem Träger getragene Zusammensetzung zwischen 10 und 1000 Gew.-%, bezogen auf das Gewicht des Trägers, ausmacht.

13. Artikel nach einem der vorhergehenden Ansprüche, **dadurch gekennzeichnet, dass** die von dem Träger getragene Zusammensetzung außerdem mindestens eine unter schaumbildenden Tensiden, Polymeren, lipophilen Verbindungen, Peelingmitteln, Wirkstoffen und Mischungen davon ausgewählte Verbindung umfasst.

14. Artikel nach einem der vorhergehenden Ansprüche, **dadurch gekennzeichnet, dass** er ein Reinigung- oder Abschminkprodukt für die Haut, ein Peelingprodukt, ein Hautpflegeprodukt, ein Pflaster zum Abspülen, ein Haarprodukt oder ein Makeup-Produkt darstellt.

15. Artikel nach einem der vorhergehenden Ansprüche, **dadurch gekennzeichnet, dass** der Träger ein Kissen, eine Maske, ein Pflaster, eine Damenhaube, einen Fingerling, einen Handschuh, eine Lage zum Zuschneiden, ein Tuch, eine Scheibe, ein Oval oder ein Rechteck bildet.

16. Zuaammensetzung zur topischen Abwendung, erhalten durch Lösen eines Artikels nach einem der Ansprüche 1 bis 15 in Wasser.

17. Verfahren zur kosmetischen Behandlung eines Keratinmaterials, bei dem man:
- eine kosmetische Zusammensetzung bildet, indem man einen Träger, der mindestens eine Lage aus bei einer Temperatur kleiner gleich 30°C wasserlöslichen Fasern umfasst, wobei der Träger eine Dichte kleiner gleich 0,1 g/cm³ aufweist und mindestens eine Verbindung mit thermischem Effekt trägt, wobei der Träger **dadurch gekennzeichnet ist, dass** er mindestens zwei Lagen umfasst, die zusammen einen Hohlraum definieren, wobei mindestens eine der Lagen bei einer Temperatur kleiner gleich 30°C wasserlösliche Fasern umfasst, und der Hohlraum die mindestens eine verbidung mit thermischem Effekt enthaltend Zusammensetzung enthält, in Wasser löst;
- die so gebildete zuaammensetzung auf das κeratinmaterial aufbringt.

18. Einheit, umfassend:
- eine Verpackung,
- mindestens einen Artikel nach einem der Ansprüche 1 bis 15.
